# EUROPÄISCHE PATENTSCHRIFT

(11) **EP 0 538 348 B1**
(45) Veröffentlichungstag und Bekanntmachung des Hinweises auf die Patenterteilung: **02.10.1996**
(21) Anmeldenummer: 91912958.5
(22) Anmeldetag: 05.07.1991
(51) Int. Cl.: C07J 41/00, A61K 31/565

(54) **UNGESÄTTIGTE 15-DEHYDRO-17alpha-CYANOMETHYL-17beta- HYDROXYSTEROIDE**
UNSATURATED 15-DEHYDRO-17alpha-CYANOMETHYL-17beta- HYDROXYSTEROIDS
15-DEHYDRO-17alpha-CYANOMETHYL-17beta-HYDROXYSTEROIDES INSATURES

(30) Priorität: 09.07.1990 DD 342604; 09.07.1990 DD 432605; 09.07.1990 DD 432603
(43) Veröffentlichungstag der Anmeldung: 28.04.1993
(73) Patentinhaber: JENAPHARM GmbH, D-07745 Jena (DE)
(72) Erfinder: TEICHMÜLLER, Gerhard, D-6900 Jena (DE); MÜLLER, Gerd, D-6900 Jena (DE)
(86) Internationale Anmeldenummer: DE9100562
(87) Internationale Veröffentlichungsnummer: WO9200991

(56) Entgegenhaltungen:
- EP-A- 0 231 671
- DD-A- 160 418
- Steroids, Vol. 39, Nr. 4, April 1982, G. Hobe et al.: "Microbial transformation of 17alpha-cyanomethyl-17-hydroxy-4,9-estradien-3-one (STS 557)17alpha- cyanomethyl-19-nortestosterone by Mycobacterium Smegmatis", Seiten 399-409, siehe das ganze Artikel
- Die Pharmazie, Band 39, Nr. 7, 1984, VEB Verlag Volk und Gesundheit, (Berlin, DE), M. Hübner et al.: "Synthese potentieller Metaboliten der STS 557 (Dienogest)", Seite 496, siehe das ganze Artikel
- Chemical Abstracts, Vol. 104, Nr. 23, 9. Juni 1986, (Columbus, Ohio, US), Q. Cheng et al.: "Synthesis of 17alpha-cyanomethyl-17beta-hydroxy-4,9(10)- estradien-3-one and its 4,9(10),11-triene derivative", siehe Seite 782, Zusammenfassung 207513f, & Yiyao Gongye 1985, 16(9), 399-402
- Die Pharmazie, Band 39, Nr. 7, 1984, VEB Verlag Volk und Gesundheit, (Berlin, DE), M. Hübner et al.: "Synthese potentieller Metaboliten der STS 557 (Dienogest) - Teil 1: Metaboliten mit einem hydrierten A/B-Ring-System", Seiten 462-463, siehe das ganze Artikel
- Die Pharmazie, Band 41, Nr. 9, 1986, VEB Verlag Volk und Gesundheit, (Berlin, DE), G. Hobe et al.: "Rinderserumalbuminkonjugate des neuen Progestagens Dienogest-Synthese und immunogene Eigenschaften", Seiten 627-630, siehe das ganze Artikel
- Chemical Abstracts, Vol. 103, Nr. 15, 14. Oktober 1985, (Columbus, Ohio, US), B Menzenbach et al. Page 755
- Chemical Abstracts, Vol. 89, Nr. 23, 4. Dezember 1978, (Columbus, Ohio, US), K. Ponsold et al.: "Steroids. 55. 17alpha-CH2X-substituted androstane and 19- norandrostane derivatives", siehe Seite 656, Zusammenfassung 197792b, & Z. Chem. 1978, 18(7), 259-60
- Tetrahedron, Vol. 28, Nr. 9, Mai 1972, Pergamon Press, G. Ferrara et al.: "Steroidal 1,2,4-oxadiazoles by 1-3 dipolar cycloaddition", Seiten 2461-2467, siehe Seite 2462, Verbindungen 4a,4d
- Studies in Organic Chemistry - Natural Products Chemistry, Poznan, 9.-14. Juli 1984, Nr. 20, Elsevier Science Publishers B.V., (Amsterdam, NL), K. Schubert et al.: "Synthesis, effects, and metabolism of the progestagen and potential interceptive dienogest", Seiten 143-158, siehe das ganze Artikel

## Beschreibung

Die Erfindung betrifft ungesättigte 15-Dehydro-17α-cyanomethyl-17β-hydroxysteroide der allgemeinen Formel I, diese enthaltende pharmazeutische Präparate sowie Verfahren zu deren Herstellung gemäß Patentansprüchen. Ungesättigte 17α-Cyanomethyl-17β-hydroxysteroidderivace sind pharmakologisch interessante Steroidverbindungen oder auch Zwischenprodukte zur Synthese pharmakologisch hochwirksamer Steroidprodukte wie z.B. 17α-Cyanomethyl-17β-hydroxy-13-alkyl-4,9-gonadien-3-on wie "Dienogest" oder auch 17α-Cyanomethyl-17β-hydroxy-13-alkyl-4,9,11-gonatrien-3-on derivate, die auf Grund ihrer spezifischen homonellen oder auch antihormonellen Wirkungen vorteilhaft zur Behandlung von Endokrinopathien und zur Reproduktionslenkung in der Human- und Veterinärmedizin eingesetzt werden können. Besonders vorteilhaft ist in der Anwendung die gute Verträglichkeit dieser Verbindungen, die auch bei erhöhter Dosierung kaum unerwünschte Nebenwirkungen hervorrufen und so auch im Vergleich zu den bekannten 17α-Ethinyl-17β-hydroxysteroiden eine wünschenswerte Bereicherung der Palette der pharmakologisch interessanten Steroidwirkstoffe darstellen.

Die Einführung einer weiteren Doppelbindung in diese 17α-Cyanomethyl-17β-hydroxysteroide in die Position C₁₅ / C₁₆ des Steroidgrundgerüstes führt zu einer signifikanten Wirkungssteigerung, wobei eine Reihe dieser neuen Derivate besonders günstige Dissoziationen der charakteristischen Partialwirkungen zeigen.

Zur Anwendung dieser neuen Verbindungen in der Fertilitätskontrolle sind Dosierungen bis zu max. 2 mg Wirkstoff / Tag vorteilhaft. Die Verabreichung dieser neuen Wirkstoffe erfolgt in Kombination mit estrogen wirksamen Steroiden wie z.B. Ethinylestradiol in den bekannten pharmazeutischen Zubereitungsformen als Tablette oder Dragee.

Ein weiteres Einsatzgebiet der neuen Wirkstoffe ist die Behandlung spezieller Erkrankungen wie z.B. die Behandlung der Endometriose. Auch hier beträgt die Dosierung bis zu 2 mg / Tag über einen Zeitraum von 4 bis 6 Monaten. Diese niedrigen Dosierungen zeigen die Überlegenheit der neuen Verbindungen besonders deutlich, da nach der bisher für diese Indikationen üblichen Behandlung mit Danazol die Dosierung 400 bis 800 mg Danazol / Tag beträgt.

Die Synthese von 17α-Cyanomethyl-17β-hydroxysteroiden wurde in einer Reihe von Patenten beschrieben. Hiernach werden 17-Ketosteroide in einer mehrstufigen Synthese in die Steroid-17α- spiro-1',2'-oxirane überführt, die dann mit Alkalicyanid zu den 17α-Cyanomethyl-17β-hydroxysteroidderivaten umgesetzt werden.

Nach K. Ponsold u.a. DD-PS 132 497 wird 3-Methoxy-13-alkylgona-2,5(10)-dien-17α-spiro-1',2'-oxiran (hergestellt nach DD-PS 80 023) durch Reaktion mit Alkalicyanid und nachfolgende Enoletherhydrolyse in das 17α-Cyanomethy1-17β-hydroxy-13-alkylgon-5(10)-en-3-on überführt. Eine Verbesserung dieser Synthese wurde von K. Ponsold u.a. in dem DD-WP 160 418 beschrieben, nach der das unbeständige Ausgangsmaterial des vorgenannten Verfahrens (DD-WP 132 497) durch 3,3-Dimethoxy-13-alkyl-gon-5(10)-en-3-on ersetzt wird, welches dann in gleicher Weise mit Trimethylsulfoniumjodid, Alkalicyanid und nachfolgender Ketalspaltung in das 17α-Cyanomechyl-17β-hydroxy-13-alkyl-gon- 5(10)-en-3-on überführt wird.

Obwohl die einzelnen Stufen dieser Synthese mit hohen Ausbeuten verlaufen, wird die Gesamtökonomie belastet durch den Einsatz teuerer Reagenzien und den hohen Arbeitsaufwand, hervorgerufen durch die aufwendigen Zwischenisolierungen.

Der Hauptnachteil dieser Verfahrensweise besteht jedoch in der mit dieser Synthese verbundenen hohen Umweltbelastung, die hervorgerufen wird durch den Einsatz von
- Trimethylsulfoniumjodid, was die Entsorgung der Abluft und des Abwassers erfordert,
- Alkalicyanid, einem Gift der Abt.1 des Giftgesetzes, was besondere Anforderungen an die Verarbeitung im technischen Maßstab stellt und eine spezielle Entsorgung der Abluft und der Abwässer erfordert.

E. Nitta u.a. beschreiben im EP 231 671 vom 19.06.86 die Synthese von 17α-Cyanomethyl-17β-hydroxy-13-alkyl-gona-4,9,11-trien-3-on-derivaten aus den entsprechenden 17-Ketosteroiden unter Nutzung der von K. Ponsold u.a. (DD-WP 160 418) beschriebenen Reaktionsfolge - 17-Ketosteroid ----> 17α-Spiro-1',2'-oxiran ---> 17α-Cyanomethyl-17β-hydroxysteroidderivate. Obwohl mit diesem Patent neue Verbindungen des 17α-Cyanomethyl-17β-hydroxyeteroidtyps beschrieben wurden, konnten keinerlei Verbesserungen des ursprünglichen Syntheseweges offenbart werden, d.h. die bereits angeführten Mängel des hohen Arbeitsaufwandes und der starken Umweltbelastungen bestehen auch bei diesem Verfahren. Zu den bereits angeführten hohen Aufwendungen für die Durchführung dieser Synthese kommen bei der technischen Durchführung die ständigen zusätzlichen hohen Aufwendungen für die Entsorgung der Abprodukte, der anfallenden Abwässer und der Abluft mit Schwefelverbindungen und Cyaniden, wodurch die Ökonomie des Gesamtverfahrens wesentliche zusätzliche Belastungen erfährt.

Ziel der Erfindung ist die Synthese von neuen pharmakologisch hochwirksamen 15-Dehydco-17α-cyano-methylverbindungen, die gegenüber den bekannten 17α-Cyanomethyl-17β-hydroxystaroid-derivaten eine signifikante Wirkungssteigerung oder weiter verbesserte Dissoziation der charakteristischen Partialwirkungen aufweisen und so auf Grund ihrer verbesserten spezifischen Wirkungen vorteilhafter zur Behandlung von Endokrinopathien und zur Reproduktionslenkung in der Human- und Veterinärmedizin eingesetzt werden können.

Ziel der Erfindung ist es auch, zur Herstellung dieser neuen hochwirksamen 15-Dehydro-17α-cyanomethylderivate synthesemethoden zu entwickeln, die einen möglichst geringen Arbeitsaufwand erfordern und Umweltbelastungen weitestgehend vermeiden.

Der Erfindung liegt die Aufgabe zu Grunde, neue pharmakologisch hochwirksame 15-Dehydro-17α-cyanomethyl-17β-hydroxysteroidderivate zu synthetisieren und ein Verfahren bereitzustellen, nach dem die Zielprodukte in technisch einfacher und ökonomisch günstiger Weise zugänglich gemacht werden können und der Aufwand für Nebenprozesse, insbesondere die Abproduktbeseitigung und Entsorgung von Abwässern und Abluft weitestgehend minimiert werden können.

Die vorliegende Erfindung betrifft ungesättigte 15-Dehydro-17α-cyanomethyl17β-hydroxysteroide der allgemeinen Formel I 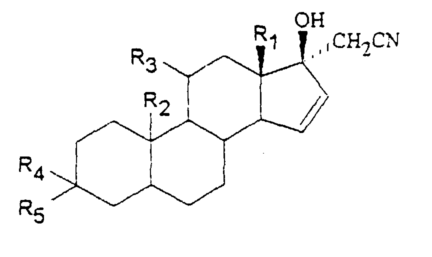 in der
R₁ = CH₃ , C₂H₅
R₂ = H,CH₃,R₂ entfällt, wenn am C₁₀ eine Doppelbindung steht,
R₃ = H,OH, O-Acyl-, O-Alkylgruppe mit 1 bis 6 C-Atomen
R₄ = H
R₅ = OH, O-Acyl-, O-Alkylgruppe mit 1 bis 6 Kohlenstoffatomen oder R₄ und R₅ zusammen Keto- oder Ketalgruppe darstellen können, wobei für die Ketalgruppe (CH₃O)₂;(CH₃CH₂O)₂;-O-CH₂-CH₂-O-;-O-CH₂-(Me)C(Me)-CH₂-O-; -OCH(Me)-CH₂-(Me)CH-O- stehen kann und im Grundgerüst Doppelbindungen in den Positionen C₁=C₂, C₂=C₃, C₃=C₄, C₄=C₅, C₅=C₆, C₅=C₁₀, C₉=C₁₀ und C₉=C₁₁ vorhanden sein können.

Nachstehende Verbindungen sind erfindungsgemäß besonders bevorzugt, 13-Methyl-17a-cyanomethyl-17b-hydroxy-5(10),15-gonadien-3-on 13-Methyl-17α-cyanomethyl-17β-hydroxy-4,15-gonadien-3-on 13-Methyl-17α-cyanomethyl-3,17β-dihydroxy-gona-1,3,5(10),15-tetraen 13-Methy1-3-methoxy-17α-cyanomethyl-17β-hydroxy-gona-1,3,5(10),15-tetraen 13-Methyl-3-methoxy-17α-cyanomethyl-17β-hydroxy-gona-3,5(10),15-trien 13-Methyl-3,3-dialkoxy-17α-cyanomethyl-17β-hydroxy-gona-5(10),15-dien 13-Methyl-3,3-ethyilendioxy-17α-cyanomethyl-17β-hydroxy-gona-5 (10), 15-dien 13-Ethyl-17α-cyanomethyl-17β-hydroxy-5(10),15-gonadien-3-on 13-Ethyl-17α-cyanomethyl-17β-hydroxy-4,15-gonadien-3-on 13-Ethyl-17α-cyanomethyl-3,17β-dihydroxy-gona-1,3,5(10),15-tetraen 13-Ethyl-3-methoxy-17α-cyanomethyl-17β-hydroxy-gona-1,3,5(10),15-tetraen 13-Ethyl-3-methoxy-17α-cyanomethyl-17β-hydroxy-gona-3,5(10),15-trien 13-Ethyl-3,3-dialkoxy-17α-cyanomethyl-17β-hydroxy-gona-5(10), 15-dien 13-Ethyl-3,3-ethylendioxy-17α-cyanomethyl-17β-hydroxy-gona-5(10),15-dien

Erfindungsgemäß wird die Aufgabe dadurch gelöst, daß ungesättigte 17-Ketosteroide der allgemeinen Formel II 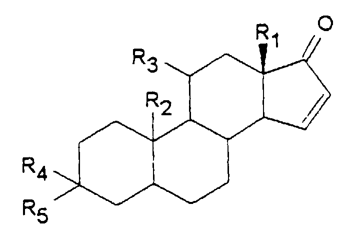 in der die Reste
R₁ = Me, Et
R₂ = Me, H, R₂ entfällt,wenn am C₁₀ eine Doppelbindung steht
R₃ = H, OH, O-Acyl mit 1 bis 6 Kohlenstoffatomen,
R₄ = H
R₅ = OH,O-Acyl-, O-Alkylgruppe mit 1 bis 6 Kohlenstoffatomen, oder R₄ und R₅ zusammen eine Ketalgruppe darstellen können, wobei für die Ketalgruppe (CH₃O)₂, (CH₃CH₂O)₂,-O-CH₂-CH₂-O-; -O-CH₂-(Me)C(Me)-CH₂-O-; -O-CH(Me)-CH₂-(Me)CH-O- stehen kann und im Grundgerüst des Steroidmoleküls Doppelbindungen in den Positionen C₁=C₂, C₂=C₃, C₃=C₄; C₄=C₅;
C₅=C₆; C₅=C₁₀; C₉=C₁₀ und C₉=C₁₁ vorhanden sein können, in einem

Eintopfverfahren in inerten organischen Lösungsmitteln bei tiefen Temperaturen mit LiCH₂CN, das in situ aus CH₃CN durch Reaktion mit Lithiumalkylen oder Lithiumdialkylamiden (Alkyl = C₁ bis C₆ ) bereitet wird, umgesetzt, die Reaktionsprodukte wäßrig aufgearbeitet, die Verbindungen der allgemeinen Formel I, in der die Reste
R₁ = Me, Et
R₂ = Me, H, R₂ entfällt,wenn am C₁₀ eine Doppelbindung steht
R₃ = H, OH, O-Acyl mit 1 bis 6 Kohlenstoffatomen,
R₄ = H
R₅ = OH,O-Acyl-, O-Alkylgruppe mit 1 bis 6 Kohlenstoffatomen, oder R₄ und R₅ zusammen eine Ketalgruppe darstellen können, wobei für die Ketalgruppe (CH₃O)₂,(CH₃CH₂O)₂, -O-CH₂-CH₂-O-; -O-CH₂-(Me)C(Me)-CH₂-O-; -O-CH(Me)-CH₂-(Me)CH-O- stehen kann und im Grundgerüst des Steroidmoleküls Doppelbindungen in den Positionen C₁=C₂, C₂=C₃, C₃=C₄; C₄=C₅;
C₅=C₆; C₅=C₁₀; C₉=C₁₀ und C₉=C₁₁ vorhanden sein können, isoliert oder durch direkte Säurehydrolyse in obengenannte Verbindungen der allgemeinen Formel I mit den Resten
R₁ = Me, Et
R₂ = Me, H, R₂ entfällt, wenn am C₁₀ eine Doppelbindung steht
R₃ = H, OH
R₄ = H,
R₅ = OH, oder
R₄ und R₅ zusammen eine Ketogruppe darstellen können, und im Grundgerüst des Steroidmoleküls Doppelbindungen in den Positionen C₁=C₂, C₂=C₃, C₃=C₄; C₄=C₅; C₅=C₆; C₅=C₁₀; C₉=C₁₀ und C₉=C₁₁ vorhanden sein können, überführt werden.

Die in situ erfolgende Metallierung des Acetonitrils mit Lithiumalkylen oder Lithiumdialkylamiden zum LiCH₂CN sowie dessen nachfolgende Umsetzung in einem Eintopfverfahren mit ungesättigten 17-Ketosteroiden zu den ungesättigten 15-Dehydro-17α-cyanomethyl-17β-hydroxysteroiden erfolgt in inerten organischen Lösungsmitteln wie aliphatischen oder aromatischen Kohlenwasserstoffen, vor-zugsweise Pentan, Hexan, Heptan, Benzen, Toluen, Ethern vorzugsweise Diethylether, Diisopropylether, Tetrahydrofuran, Dioxan, Anisol, Methyl-t-butylether Dimethoxyethan, Diethoxyethan oder auch in tertiären Aminen, wie z.B. Triethylamin, Diisopropylethylamin, Pyridin, Tetraalkylethylendiamin, oder diese Lösungsmittel können auch als Gemisch verwendet werden.

Nach erfindungsgemäßer Verfahrensweise werden pro Mol unges. 17-Ketosteroid 1 bis 5 Mol Acetonitril und 1 bis 5 Mol Lithiumalkyl bzw. Lithiumdialkylamid eingesetzt, wobei so verfahren wird, daß das Acetonitril bei tiefen Temperaturen mit den eingesetzten Lithiumderivaten in situ in das LiCH₂CN überführt und dieses mit dem ungesättigtem 17-Ketosteroid zum ungesättigtem 15-Dehydro-17α-cyanomethyl-17β-hydroxysteroid umgesetzt wird.

Diese Verfahrensweise schließt auch ein, daß die in-situ-Darstellung des LiCH₂CN in Gegenwart des ungesättigten 17-Ketosteroids erfolgt und die Nachfolgereaktion mit dem ungesättigtem 17- Ketosteroid zum ungesättigten 15-Dehydro-17α-cyanomethyl-17β-hydroxyderivat nahezu gleichzeitig ablaufen kann.

Diese Verfahrensweise schließt auch ein, daß die Reaktionen des Alkyllithiums mit dem Acetonitril auch in Gegenwart von Lithiumhalogeniden durchgeführt werden kann, d.h. daß die Abtrennung des Li-Hal vor der weiteren Verwendung des Alkyllithiums nicht erforderlich ist bzw. die Gegenwart von Lithiumhalogenid die Durchführung der Folgereaktionen nicht stört. Vorteilhaft werden diese Reaktionen bei tiefen Temperaturen durchgeführt, d.h. in Temperaturbereichen unter 0 °C, bei denen die Reaktion des LiCH₂CN mit Acetonitril zum β-Kecopropionitril 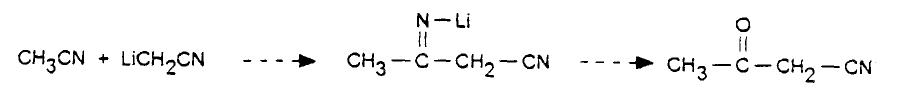 weitestgehend bzw. vollständig unterdrückt wird. Vorzugsweise sind das die Temperaturbereiche von -20 °C bis -90 °C. Die Aufarbeitung der Reaktionsgemische kann durch Zusatz von Wasser erfolgen, wobei die Reaktionsprodukte unter Erhalt von Schutzgruppen wie Ketalen oder Enolethern u.a. isoliert werden können. Diese wäßrigen Aufarbeitungen erfolgen vorteilhaft bei Temperaturen ≤10 °C in pH-Bereichen ≥ pH 6, wobei die pH-einstellung auch durch Zusätze von Salzen wie NH₄Cl, NH₄OAc, NaH₂PO₄ oder auch Säuren erfolgen kann.

Die Aufarbeitung kann aber auch so geführt werden, daß die Zersetzung der Reaktionsgemische, d,h. die Zerstörung des überschüssigen Lichiumorganyls unter Zusatz von Wasser und Säuren bzw. verdünnten Säuren erfolgt und bei pH-Bereichen ≤ pH 6 durchgeführt wird und hierbei gegebenenfalls im Molekül vorhandene Schutzgruppierungen wie Ketale, Enolether u.a. gespalten werden. Als Säuren können hier anorganische Säuren wie z.B. HCl, E₃PO₄ , H₂SO₄ u.a. oder auch organische Säuren wie z.B. Essigsäure, Oxalsäure, p-Toluensulfonsäure u.a. eingesetzt werden.

Bei der Aufarbeitung kann allgemein so verfahren werden, daß die Zersetzung des überschüssigen Li-Organyls bei tiefen Temperaturen begonnen und die Säurehydrolyse dann bei Temperaturen bis zu +50 °C weitergeführt werden kann. Die Isolierung der Reaktionsprodukte kann durch Extraktion oder Fällung erfolgen, vorteilhaft ist es jedoch, vom Reaktionsgemisch unter Vakuum das Lösungsmittelgemisch weitestgehend abzudestillieren und die hierbei, oder durch Zusatz von einem Alkohol oder Keton in hoher Reinheit anfallenden Kristalle zu isolieren.

Unter Anwendung der neuen Verfahrensweise, der einstufigen Einführung der Cyanomethylgruppe in die 17α-Stellung des Steroidmoleküls wurden Voraussetzungen für eine technisch durchführbare, umweltschonende Synthese geschaffen. Mit dieser neuen Verfahrensweise werden die bekannten Umweltbelastungen vermieden, da die Lösungsmittel weitestgehend zurückgewonnen, das bei der Aufarbeitung anfallende Lithiumsalz gut abgetrennt werden kann und die im Abwasser enthaltenen Restmengen an Acatonitril in verdünnten Lösungen biologisch abbaufähig sind (Martinez Immobilisation, Entgiftung und Zerstörung von Chemikalien", 1.Aufl., Verlag Harri Deutsch, Thun-Frankfurt/ Main, S.211).

### Beispiel 1

### 3-Methoxy-13-ethyl-17α-cyanomethyl-17β-hydroxy-3,5,15-gonatrien

Unter Inertgas gibt man 9,5 ml Butyllithiumlösung (14,5 mmol Butyl-lithium) in ein Reaktionsgefäß, kühlt auf Temperaturen ≤-60 °C und versetzt dann mit 10,0 ml gereinigtem, trockenen Terahydrofuran. Zu dieser Lösung gibt man unter Rühren und Kühlen 0,78 ml Acetonitril (15 mmol). Anschließend wird eine Lösung von 2 g 3-Methoxy-13-ethyl-3,5,15-gonatrien-17-on (6,7 mmol) in 10 ml Tetrahydrofuran so zugesetzt, daß die Reaktionstemperatur von -60 °C nicht überschritten wird. Nach Zugabe der Steroidlösung läßt man noch bis zu 1 Stunde nachreagieren, läßt dabei das Reaktiosgemisch bis auf etwa -10 °C erwärmen und versetzt bei dieser Temperatur beginnend tropfenweise mit 10 ml Wasser, wobei die Temperatur des Reaktionsgemisches bis auf +10 °C ansteigen kann. Die Phasen werden getrennt, die org. Phase eingeengt und der Rückstand mit Methanol zur Kristallisation gebracht.
Ausbeute:1,8 g = 79 % d.Th.
Fp: 195,2 °C bis 197,5 °C
¹H-NMR-Spektrum: (80 MHz, CDCl₃ , δ gegen TMS als inneren Standard) 0,90 ppm (tr, 3H, J = 7 Hz, 13-CH₂ CH₃); 2,54 ppm (s, 2H, 17α-CH₂ CN); 3,28 ppm (s, 3H, 3-OCH₃ ); 5,23 ppm (s, 1H, 4-CH); 5,30 ppm (m, 1H, 6-CH); 5,74 ppm (dd, 1H, J = 6 Hz u. 3 Hz, 15- CH); 6,00 ppm (d, 1H, J = 6 Hz, 16-CH).

### Beispiel 2

### 13-Ethyl-17α-cyanomethyl-17β-hydroxy-4,15-gonadien-3-on

Nach der im Beispiel 1 beschriebenen Verfahrensweise werden 2 g 3-Methoxy-13-ethyl-3,5,15-gonatrien-17-on bei Reaktionstemperaturen ≤ -35 °C zum 3-Methoxy-13-ethyl-17α-cyanomethyl-17β-hydroxy-3,5,15-gonatrien umgesezt. Nach Ablauf der Nachreaktion wird das Reaktions-gemisch bei Temperaturen von etwa -15 °C beginnend mit verdünnter Schwefelsäure versetzt, wobei die Temperatur des Reaktionsgemisches bis auf 25 °C ansteigen kann. Nach Abtrennung der wäßrigen Phase wird die organische Phase unter Vakuum eingeengt und unter Zusatz von Methanol zur Kristallisation gebracht. Man läßt nach erfolgter Kristallisation noch bis zu 1 Stunde rühren, kühlt dann auf etwa 10 °C ab und saugt das Kristallisat ab.
¹H-NMR-Spektrum; (80 MHz, CDCl₃ , δ gegen TMS als inneren Standard) 0,94 ppm (tr, 3H, J = 7 Hz, 18-CH₂ CH₃ ); 2,55 ppm (s, 17α-CH₂ CN); 5,78 ppm (dd, 1M, J = 6 und 3 Hz, 15-CH); 5,89 ppm (s, 1H, 4-CH); 6,03 ppm (d, 1H, J = 6 Hz, 16-CH)

### Beispiel 3

### 3-Methoxy-17α-cyanomethyl-17β-hydroxy-1,3,5(10),15-estratetraen

27,7 ml Butyllithiumlösung (0,0443 mol) werden unter Inertgas in einem Sulfierkolben gegeben und unter Rühren auf -60 °C abgekühlt. Bei dieser Temperatur werden nacheinander 15 ml Tetrahydrofuran und 2,5 ml acetonitril so zugesetzt, daß die Temperatur des Reaktionsgemisches -60 °C nicht übersteigt. 5,0 g 15-Dehydro-estron-3-methylether werden in 20 ml Tetrahydrofuran gelöst bzw. suspendiert und so zum Reaktionsgemisch gegeben, daß während der Zugabe die Temperatur des Reaktionsgemisches - 40 °C nicht übersteigt. Nach Zugabe des Steroidgemisches läßt man bei der genannten Temperatur noch bis zu 1 Stunde nachreagieren, läßt dann auf -20 °C erwärmen und zersetzt dann das Reaktionsgemisch durch Zusatz von 20 ml Wasser und stellt durch Zusatz von verdünnter Schwefelsäure auf den Neu tralpunkt ein. Nach der Phasentrennung wird die organische Phase unter Vakuum eingeengt. Der erhaltene Rückstand wird aus Methanol zur Kristallisation gebracht und isoliert.
Ausbeute: 4,5 g
¹H-NMR-Spektrum: (80 MHz, CDCl₃, δ gegen TMS als inneren Standard) 0,97 ppm (s,3H,18-CH₃ ); 2,58 ppm (d, 2H, 17α-CH₂CN); 3,80 ppm (s, 3H, 3-OCH₃ ); 5,32 ppm (dd, 1H, J = 6 und 3Hz, 15-CH); 6,16 ppm (d, 1H, J = 6Hz, 16-CH); 6,68 ppm (m, 1H, 4-CH); 6,78 ppm (dd, 1H, 2-CH); 7,21 ppm (d, 1H,1-CH)

### Beispiel 4

### 13-Ethyl-17α-cyanomethyl-17β-hydroxy-4,15-gonadien-3-on

Nach der im Beispiel 1 beschriebenen Verfahrensweise werden 4,0 g 3,3-(Propylen-1,3-dioxy)-13-ethyl-5,15-gonadien-17-on mit einem Gemisch aus Butyllithium, Tetrahydrofuran und Acetonitril umgesetzt bei Temperaturen <-35 °C. Nach saurer Hydrolyse und Extraktion mit CHCl₃ werden 2,9 g Kristallisat isoliert.

## Patentansprüche

1. Ungesättigte 15-Dehydro-17α-cyanomethyl-17β-hydroxysteroide der allgemeinen Formel I 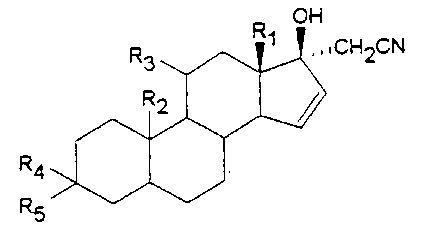 in der
R₁ = CH₃, C₂H₅
R₂ = H,CH₃,R₂ entfällt, wenn am C₁₀ eine Doppelbindung steht,
R₃ = H,OH, O-Acyl-, O-Alkylgruppe mit 1 bis 6 C-Atomen
R₄ = H
R₅ = OH,O-Acyl-, O-Alkylgruppe mit 1 bis 6 Kohlenstoffatomen oder R₄ und R₅ zusammen Keto- oder Ketalgruppe darstellen können, wobei für die Ketalgruppe (CH₃O)₂;(CH₃CH₂O)₂;-O-CH₂-CH₂-O-;-O-CH₂-(Me)C(Me)-CH₂-O-; -OCH(Me)-CH₂-(Me)CH-O- stehen kann und im Grundgerüst Doppelbindungen in den Positionen C₁=C₂, C₂=C₃, C₃=C₄, C₄=C₅, C₅=C₆, C₅=C₁₀, C₉=C₁₀ und C₉=C₁₁ vorhanden sein können.

2. 13-Methyl-17α-cyanomethyl-17β-hydroxy-5(10),15-gonadien-3-on

3. 13-Methyl-17α-cyanomethyl-17β-hydroxy-4,15-gonadien-3-on

4. 13-Methyl-17α-cyanomethyl-3,17β-dihydroxy-gona-1,3,5(10),15- tetraen

5. 13-Methyl-3-methoxy-17α-cyanomethyl-17α-hydroxy-gona-1,3,5 (10),15-tetraen

6. 13-Methyl-3-methoxy-17α-cyanomethyl-17β-hydroxy-gona-3,5(10),15-trien

7. 13-Methyl-3,3-dialkoxy- 17α-cyanomethyl- 17β-hydroxy-gona-5(10),15-dien

8. 13-Methyl-3,3-ethylendioxy-17α-cyanomethyl-17β-hydroxy-gona-5(10),15-dien

9. 13-Ethyl-17α-cyanomethyl-17β-hydroxy-5(10),15-gonadien-3-on

10. 13-Ethyl-17α-cyanomethyl-17β-hydroxy-4,15-gonadien-3-on

11. 13-Ethyl-17α-cyanomethyl-3,17β-dihydroxy-gona-1,3,5(10),15-tetraen

12. 13-Ethyl-3-methoxy- 17α-cyanomethyl-17β -hydroxy-gona-1,3,5(10),15-tetraen

13. 13-Ethyl-3-methoxy-17α-cyanomethyl-17β-hydroxy-gona-3,5(10),15-trien

14. 13-Ethyl-3,3-dialkoxy-17α-cyanomethyl-17β-hydroxy-gona-5(10),15-dien

15. 13-Ethyl-3,3-ethylendioxy-17α-cyanomethyl-17β-hydroxy-gona-5(10),15-dien

16. Pharmazeutische Präparate, gekennzeichnet durch einen Gehalt an Verbindungen gemäß Anspruch 1 bis 15

17. Verfahren zur Herstellung von ungesättigten 17α-Cyanomethyl-17β-hydroxysteroiden der allgemeinen Formel I, dadurch gekennzeichnet, daß ungesättigte 17-Katosteroide der allgemeinen Formel II, 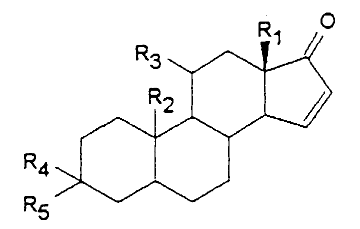 in der
R₁ = CH₃ , C₂H₅
R₂ = H,CH₃,R₂ entfällt, wenn am C₁₀ eine Doppelbindung steht,
R₃ = H,OH, O-Acyl-, O-Alkylgruppe mit 1 bis 6 C-Atomen
R₄ = H
R₅ = OH, O-Acyl-, O-Alkylgruppe mit 1 bis 6 Kohlenstoffatomen oder R₄ und R₅ zusammen eine Ketalgruppe darstellen können, wobei für die Ketalgruppe (OH₃O)₂; (CH₃CH₂O)₂;-O-CH₂-CH₂-O-;-O-CH₂-(Me)C(Me)-CH₂-O-; -OCH(Me)-CH₂-(Me)CH-O- stehen kann und im Grundgerüst Doppelbindungen in den Positionen C₁=C₂, C₂=C₃, C₃=C₄, C₄=C₅, C₅=C₆, C₅=C₁₀, C₉=C₁₀ und C₉=C₁₁ vorhanden sein können,
in einem Eintopfverfahren in inerten organischen Lösungsmitteln bei tiefen Temperaturen mit Li-CH₂CN, das in situ aus CH₃CN durch Reaktion mit Lithiumalkylen oder Lithiumdialkylamiden (Alkyl = C₁ bis C₆) bereitet wird, umgesetzt und die Reaktionsprodukte wäßrig aufgearbeitet, die Verbindungen der allgemeinen Formel I mit den Resten
R₁ = CH₃, C₂H₅
R₂ = H,CH₃,R₂ entfällt, wenn am C₁₀ eine Doppelbindung steht,
R₃ = H,OH, O-Acyl-, O-Alkylgruppe mit 1 bis 6 C-Atomen
R₄ = H
R₅ = OH, O-Acyl-, O-Alkylgruppe mit 1 bis 6 Kohlenstoffatomen oder R₄ und R₅ zusammen eine Ketalgruppe darstellen können, wobei für die Ketalgruppe (CH₃O)₂; (CH₃CH₂O)₂;-O-CH₂-CH₂-O-;-O-CH₂-(Me)C(Me)-CH₂-O-; -OCH(Me)-CH₂-(Me)CH-O- stehen kann und im Grundgerüst Doppelbindungen in den Positionen C₁=C₂, C₂=C₃, C₃=C₄, C₄=C₅, C₅=C₆, C₅=C₁₀, C₉=C₁₀ und C₉=C₁₁ vorhanden sein können, isoliert werden
oder durch direkte Säurehydrolyse in Verbindungen der Formel I überführt, in der die Reste
R₁ = CH₃ , C₂H₅
R₂ = H,CH₃,R₂ entfällt, wenn am C₁₀ eine Doppelbindung steht,
R₃ = H,OH
R₄ = H
R₅ = OH oder
R₄ und R₅ zusammen eine Ketogruppe darstellen und im Grundgerüst Doppelbindungen in den Positionen C₁=C₂, C₂=C₃, C₃=C₄, C₄=C₅, C₅=C₆, C₅=C₁₀, C₉=C₁₀ und C₉=C₁₁ vorhanden sein können.

18. Verfahren nach Anspruch 17, dadurch gekennzeichnet, daß als inerte organische Lösungsmittel aliphatische oder aromatische Kohlenwasserstoffe, vorzugsweise Pentan, Hexan, Heptan, Benzen, Toluen , Ether, vorzugsweise Diethylether, Diisopropylether, Tetrahydrofuran, Dioxan, Anisol, Dimethoxyethan, Diethoxyethan, Methyl-t.-butylether, oder auch tertiäre Amine wie z.B. Triethylamin, Diisopropylamin, Pyridin, Tetraalkylethylendiamin eingesetzt werden oder diese Lösungsmittel auch als Gemische verwendet werden.

19. Verfahren nach Anspruch 17 und 18 , dadurch gekennzeichnet, daß pro Mol der Verbindung II, in der die Reste
R₁ = CH₃ , C₂H₅
R₂ = H,CH₃,R₂ entfällt, wenn am C₁₀ eine Doppelbindung steht,
R₃ = H,OH, O-Acyl-, O-Alkylgruppe mit 1 bis 6 C-Atomen
R₄ = H
R₅ = OH, O-Acyl-, O-Alkylgruppe mit 1 bis 6 Kohlenstoffatomen oder R₄ und R₅ zusammen eine Ketalgruppe darstellen können, wobei für die Ketalgruppe (CH₃O)₂; (CH₃CH₂O)₂;-O-CH₂-CH₂-O-;-O-CH₂-(Me)C(Me)-CH₂-O-; -OCH(Me)-CH₂-(Me)CH-O- stehen kann und im Grundgerüst Doppelbindungen in den Positionen C₁=C₂, C₂=C₃, C₃=C₄, C₄=C₅, C₅=C₆, C₅=C₁₀, C₉=C₁₀ und C₉=C₁₁ enthalten sein können, 1 bis 5 Mol Acetonitril und 1 bis 5 Mol Lithiumalkyl bzw. Lithiumdialkylamid eingesetzt werden und das Acetonitril bei tiefen Temperaturen in situ zum LiCH₂CN umgesetzt wird.

20. Verfahren nach Anspruch 17 bis 19, dadurch gekennzeichnet, daß die Reaktion bei Temperaturen ≤0 °C, vorzugsweise im Temperaturbereich von -20 °C bis -90 °C durchgeführt wird.

21. Verfahren nach Anspruch 17 bis 20 , dadurch gekennzeichnet, daß die wäßrige Aufarbeitung vorzugsweise bei Temperaturen von ≤10 °C und im pH Bereich von ≥ pH 6 durchgeführt wird.

22. Verfahren nach Anspruch 17 bis 21, dadurch gekennzeichnet, daß die Säurehydrolyse im pH-Bereich ≤ pH 6 und bei Temperaturen bis +50 °C durchgeführt wird.

23. Verfahren nach Anspruch 17 bis 22, dadurch gekennzeichnet, daß die pH-Bereichseinstellung durch Zusatz von Salzen wie z.B NH₄Cl, NH₄OAc, NaH₂PO₄ oder durch Zusatz von anorganischen Säuren wie z.B.HCl, H₃PO₄, H₂SO₄, HClO₄ oder organischen Säuren wie z.B. Essigsäure, Oxalsäure, Toluolsulfonsäure zum Aufarbeitungsgemisch erfolgt.

## Claims

1. Unsaturated 15-dehydro-17α-cyanomethyl-17β-hydroxysteroids of the general formula I 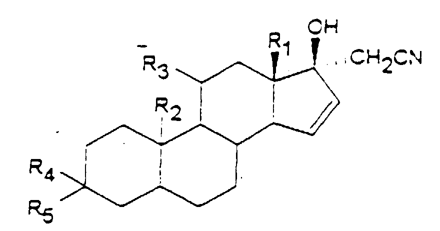 wherein
R₁ = CH₃, C₂H₅
R₂ = H, CH₃, R₂ is inapplicable when there is a double bond at C₁₀,
R₃ = H, OH, O-acyl group, O-alkyl group having 1 to 6 carbon atoms
R₄ = H
R₅ = OH, O-acyl group, O-alkyl group having 1 to 6 carbon atoms or
R₄ and R₅ together can represent a keto or ketal group, wherein the ketal group can be (CH₃O)₂; (CH₃CH₂O)₂;
-O-CH₂-CH₂-O-; -O-CH₂-(Me) C(Me) -CH₂-O- ; -OCH(Me)-CH₂-(Me)CH-O- and in the parent structure double bonds can be present in the positions C₁=C₂, C₂=C₃, C₃=C₄, C₄=C₅, C₅=C₆, C₅=C₁₀, C₉=C₁₀ and C₉=C₁₁.

2. 13-methyl-17α-cyanomethyl-17β-hydroxy-5(10),15-gonadien-3-one

3. 13-methyl-17α-cyanomethyl-17β-hydroxy-4,15-gonadien-3-one

4. 13-methyl-17α-cyanomethyl-3,17β-dihydroxygona-1,3,5(10),15-tetraene

5. 13-methyl-3-methoxy-17α-cyanomethyl-17β-hydroxygona-1,3,5(10),15-tetraene

6. 13-methyl-3-methoxy-17α-cyanomethyl-17β-hydroxygona-3,5(10),15-triene

7. 13-methyl-3,3-dialkoxy-17α-cyanomethyl-17β-hydroxygona-5(10),15-diene

8. 13-methyl-3,3-ethylenedioxy-17α-cyanomethyl-17β-hydroxygona-5(10),15-diene

9. 13-ethyl-17α-cyanomethyl-17β-hydroxy-5(10),15-gonadien-3-one

10. 13-ethyl-17α-cyanomethy1-17β-hydroxy-4,15-gonadien-3-one

11. 13-ethyl-17α-cyanomethyl-3,17β-dihydroxygona-1,3,5(10),15-tetraene

12. 13-ethyl-3-methoxy-17α-cyanomethyl-17β-hydroxygona-1,3,5(10),15-tetraene

13. 13-ethyl-3-methoxy-17α-cyanomethy1-17β-hydroxygona-3,5(10),15-triene

14. 13-ethyl-3,3-dialkoxy-17α-cyanomethyl-17β-hydroxygona-5(10),15-diene

15. 13-ethyl-3,3-ethylenedioxy-17α-cyanomethyl-17β-hydroxygona-5(10),15-diene

16. Pharmaceutical preparation, characterised by having a content of compounds according to claims 1 to 15.

17. Method for the preparation of unsaturated 17α-cyanomethyl-17β-hydroxysteroids of the general formula I, characterised in that unsaturated 17-ketosteroids of the general formula II, 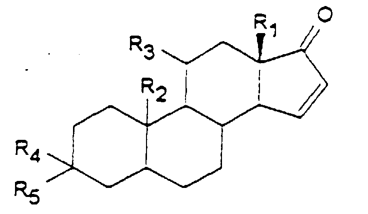 wherein
R₁ = CH₃, C₂H₅
R₂ = H, CH₃, R₂ is inapplicable when there is a double bond at C₁₀,
R₃ = H, OH, O-acyl group, O-alkyl group having 1 to 6 carbon atoms
R₄ = H
R₅ = OH, O-acyl group, O-alkyl group having 1 to 6 carbon atoms or
R₄ and R₅ together can represent a ketal group, wherein the ketal group can be (CH₃O)₂; (CH₃CH₂O)₂ ;
-O-CH₂-CH₂-O-; -O-CH₂-(Me)C(Me)-CH₂-O-; -OCH(Me)-CH₂-(Me)CH-O- and in the parent structure double bonds can be present in the positions C₁=C₂, C₂=C₃, C₃=C₄, C₄=C₅, C₅=C₆, C₅=C₁₀, C₉=C₁₀ and C₉=C₁₁,
are reacted, in a one-pot process in inert organic solvents at low temperatures, with Li-CH₂CN which is prepared in situ from CH₃CN by reaction with lithium alkyls or lithium dialkyl amides (alkyl = C₁ to C₆), and the reaction products are worked up in an aqueous medium, the compounds of the general formula I possessing the radicals
R₁ = CH₃, C₂H₅
R₂ = H, CH₃, R₂ is inapplicable when there is a double bond at C₁₀,
R₃ = H, OH, O-acyl group, O-alkyl group having 1 to 6 carbon atoms
R₄ = H
R₅ = OH, O-acyl group, O-alkyl group having 1 to 6 carbon atoms or
R₄ and R₅ together can represent a ketal group, wherein the ketal group can be (CH₃O)₂; (CH₃CH₂O)₂;
-O-CH₂-CH₂-O-; -O-CH₂-(Me)C(Me)-CH₂-O-; -OCH(Me)-CH₂-(Me)CH-O- and in the parent structure double bonds can be present in the positions C₁=C₂, C₂=C₃, C₃=C₄, C₄=C₅, C₅=C₆, C₅=C₁₀, C₉=C₁₀ and C₉=C₁₁, are isolated or converted by direct acid hydrolysis into compounds of the formula I, wherein the radicals
R₁ = CH₃, C₂H₅
R₂ = H, CH₃, R₂ is inapplicable when there is a double bond at C₁₀,
R₃ = H, OH
R₄ = H
R₅ = OH or
R₄ and R₅ together represent a keto group and in the parent structure double bonds can be present in the positions C₁=C₂, C₂=C₃, C₃=C₄, C₄=C₅, C₅=C₆, C₅=C₁₀, C₉=C₁₀ and C₉=C₁₁.

18. Method according to claim 17, characterised in that as inert organic solvents are used aliphatic or aromatic hydrocarbons, preferably pentane, hexane, heptane, benzene, toluene, ether, preferably diethyl ether, diisopropyl ether, tetrahydrofuran, dioxane, anisole, dimethoxyethane, diethoxyethane, methyl t-butyl ether, or even tertiary amines such as, for example, triethylamine, diisopropylamine, pyridine, tetraalkylethylenediamine, or these solvents are also used as mixtures.

19. Method according to claims 17 and 18, characterised in that per mol of compound II, wherein the radicals
R₁ = CH₃, C₂H₅
R₂ = H, CH₃, R₂ is inapplicable when there is a double bond at C₁₀,
R₃ = H, OH, O-acyl group, O-alkyl group having 1 to 6 carbon atoms
R₄ = H
R₅ = OH, O-acyl group, O-alkyl group having 1 to 6 carbon atoms or
R₄ and R₅ together can represent a ketal group, wherein the ketal group can be (CH₃O)₂; (CH₃CH₂O)₂;
-O-CH₂-CH₂-O-; -O-CH₂-(Me)C(Me)-CH₂-O-; -OCH (Me)-CH₂-(Me)CH-O- and the parent structure can contain double bonds in the positions C₁=C₂, C₂=C₃, C₃=C₄, C₄=C₅, C₅=C₆, C₅=C₁₀, C₉=C₁₀ and C₉=C₁₁,
1 to 5 mol of acetonitrile and 1 to 5 mol of lithium alkyl or lithium dialkylamide are added and the acetonitrile is reacted in situ at low temperatures to form LiCH₂CN.

20. Method according to claims 17 to 19, characterised in that the reaction is carried out at temperatures of ≤0°C, preferably in the temperature range of from -20°C to -90°C.

21. Method according to claims 17 to 20, characterised in that the aqueous working up is carried out preferably at temperatures of ≤10°C and in the pH range of ≥ pH 6.

22. Method according to claims 17 to 21, characterised in that the acid hydrolysis is carried out in the pH range of ≤ pH 6 and at temperatures of up to +50°C.

23. Method according to claims 17 to 22, characterised in that the pH range is adjusted by the addition of salts such as, for example, NH₄Cl, NH₄OAc, NaH₂PO₄, or by the addition of inorganic acids such as, for example, HCl, H₃PO₄, H₂SO₄, HClO₄ or of organic acids such as, for example, acetic acid, oxalic acid, toluenesulphonic acid, to the mixture being worked up.

## Revendications

1. 15-déhydro-17α-cyanométhyl-17β-hydroxystéroides insaturés de la formule générale I : 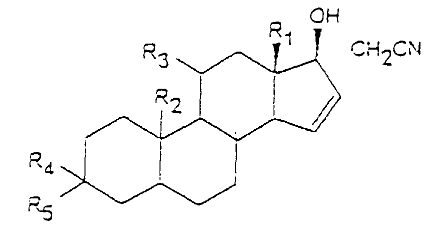 dans laquelle
R₁ = CH₃ , C₂H₅
R₂ = H, CH₃, R₂ s'annule quand C₁₀ présente une double liaison,
R₃ = H, OH, O-acyle-, groupe O-alkyle avec 1 à 6 atomes de carbone
R₄ = H
R₅ = OH, O-acyle, groupe O-alkyle avec 1 à 6 atomes d'azote ou
R₄ et R₅ ensemble peuvent représenter le groupe cétonique ou cétale, pour le groupe cétale (CH₃O)₂ ; (CH₃CH₂O)₂ ;
-O-CH₂-CH₂-O- ; -O-CH₂-(Me)C(Me)-CH₂-O-; -OCH(Me)-CH₂-(Me)CH-O- pouvant être en position verticale et dans l'ossature de base, des doubles liaisons peuvent être présentes dans les positions C₁=C₂, C₂=C₃, C₃=C₄, C₄=C₅, C₅=C₆, C₅=C₁₀, C₉=C₁₀ et C₉=C₁₁.

2. 13-méthyl-17α-cyanonéthyl-17β-hydroxy-5(10), 15-gonado-3-on

3. 13-méthyl-17α-cyanométhyl-17β-hydroxy-4, 15-gonado-3-on

4. 13-méthyl-17α-cyanométhyl-3, 17β-dihydroxy-gona-1,3,5(10), 15-tétraène.

5. 13-méthyl-3-methoxy-17α-cyanométhyl-17β-hydroxy-gona-1,3,5 (10), 15-tétraène.

6. 13-méthyl-3-methoxy-17α-cyanométhyl-17 β-hydroxy-gona-3,5(10), 15-triène

7. 13-méthyl-3,3-dialkoxy-17α-cyanométhyl-17 β-hydroxy-gona-5(10), 15-diène

8. 13-méthyl-3,3-éthylendioxy-17α-cyanométhyl-17 β-hydroxy-gona-5(10), 15-diène

9. 13-éthyl-17α-cyanométhyl-17β-hydroxy-5(10), 15-gonado-3-on

10. 13-éthyl-17α-cyanométhyl-17β-hydroxy-4, 15-gonado-3-on

11. 13-éthyl-17α-cyanométhyl-3, 17 β-dihydroxy-gona-1,3,5(10), 15-tétraène

12. 13-éthyl-3-méthoxy-17α-cyanométhyl-17 β-hydroxy-gona-1,3,5(10), 15-tétraène

13. 13-éthyl-3-méthoxy-17α-cyanométhyl-17 β-hydroxy-gona-3,5(10), 15-triène

14. 13-éthyl-3,3-dialkoxy-17α-cyanomethyl-17 β-hydroxy-gona-5(10), 15-diène

15. 13-éthyl-3,3-éthyiendioxy-17α-cyanométhyl-17 β-hydroxy-gona-5(10), 15-diène

16. Préparations pharmaceutiques, caractérisées par une teneur en composés d'après les revendications 1 à 15.

17. Procédé pour la fabrication de 17α-cyanométhyl-l7β-hydroxystéroides insaturés de la formule générale I caractérisé en ce que les 17-kétostéroïdes insaturés de la formule générale II : 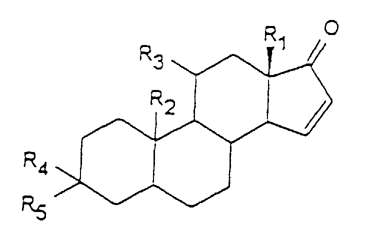 dans laquelle
R₁ = CH₃ , C₂H₅
R₂ = H, CH₃, R₂ s'annule quand C₁₀ présente une double liaison,
R₃ = H, OH, O-acyle-, groupe O-alkyle avec 1 à 6 atomes de carbone
R₄ = H
R₅ = OH, O-acyle, groupe O-alkyle avec 1 à 6 atomes d'azote ou R₄ et R₅ ensemble peuvent représenter un groupe cétale, pour le groupe cétale (CH₃O)₂ ; (CH₃CH₂O)₂ ; -O-CH₂-CH₂-O- ;
-O-CH₂-(Me)C(Me)-CH₂-O-; -OCH(Me)-CH₂-(Me)CE-O- pouvant être en position verticale et dans l'ossature de base, des doubles liaisons peuvent être présentes dans les positions C₁=C₂, C₂=C₃, C₃=C₄, C₄=C₅, C₅=C₆, C₅=C₁₀, C₉=C₁₀ et C₉-C₁₁,
sont transformés, dans un procédé à monocreuset, dans des solutions organiques inertes à de basses températures avec du Li-CH₂CN, qui est préparé in situ à partir de CH₃ CN par réaction avec des alkyles ou dialylamides de lithium (alkyle = C₁ à C₆), et les produits réactifs sont traités de manière aqueuse, les liaisons de la formule générale I sont isolées avec les restes
R₁ = CH₃, C₂H₅
R₂ = H, CH₃, R₂ s'annule quand C₁₀ présente une double liaison,
R₃ = H, OH, O-acyle-, groupe O-alkyle avec 1 à 6 atomes de carbone
R₄ = H
R₅ = OH, O-acyle, groupe O-Alkyle avec 1 à 6 atomes d'azote ou R₄ et R₅ ensemble peuvent représenter le groupe cétale, pour le groupe cétale (CH₃O)₂ ; (CH₃CH₂O)₂ ; -O-CH₂-CH₂-O- ;
-O-CH₂-(Me)C(Me)-CH₂-O- ; -OCH-(Me)-CH₂-(Me)CH-O- pouvant être en position verticale et dans l'ossature de base, des doubles liaisons peuvent être présentes dans les positions C₁=C₂, C₂=C₃, C₃=C₄, C₄=C₅, C₅=C₆, C₅=C₁₀, C₉=C₁₀ et C₉=C₁₁ ou sont transformées par acidolyse directe en composés de la formule I dans laquelle les restes
R₁ = CH₃ , C₂H₅
R2 = H, CH₃, R₂ s'annule quand C₁₀ présente une double liaison,
R₃ = H, OH,
R₄ = H
R₅ = OH, ou R₄ et R5 ensemble peuvent représenter un groupe cétonique, dans l'ossature de base, des doubles liaisons peuvent être présentes dans les positions C₁=C₂, C₂=C₃, C₃=C₄, C₄=C₅, C₅=C₆, C₅=C₁₀, C₉=C₁₀ et C₉=C₁₁.

18. Procédé selon la revendication 17, caractérisé en ce qu'en tant que solution organique inerte, on utilise des hydrocarbures aromatiques ou aliphatiques, de préférence du du pentane, de l'hexane, de l'heptane, du benzène, du toluène, de l'éther, de préférence du diéthyléther, du diisopropyléther, du tétrahydrofurane, du dioxane, de l'anisol, du diméthoxyéthane, du diéthoxyéthane, du méthyl-t.-butyléther, ou également des amines tertiaires comme par exemple du triéthylamine, du diisopropylamine, du pyridine, du tétraalkyléthylendiamine ou ses solutions peuvent également etre utilisées sous forme de mélange.

19. Procédé selon la revendication 17 et 18, caractérisé en ce que par mol du composé II, dans lequel les restes
R₁ = CH₃ , C₂H₅
R₂ = H, CH₃, R₂ s'annule quand C₁₀ présente une double liaison,
R₃ = H, OH, O-acyle-, groupe O-alkyle avec 1 à 6 atomes de carbone
R₄ = H
R₅ = OH, 0-acyle, groupe O-alkyle avec 1 à 6 atomes d'azote ou R₄ et R₅ ensemble peuvent représenter un groupe cétale, pour le groupe cétale (CH₃O)₂ ; (CH₃CH₂O)₂ ; -O-CH₂-CH₂-O- ;
-O-CH₂-(Me)C(Me)-CH₂-O-; -OCH(Me)-CH₂-(Me)CH-O- pouvant être en position verticale et dans l'ossature de base, des doubles liaisons peuvent être présentes dans les positions C₁=C₂, C₂=C₃, C₃=C₄, C₄=C₅, C₅=C₆, C₅=C₁₀, C₉=C₁₀ et C₉=C₁₁, 1 à 5 mol d'acétonitrure et 1 à 5 mol d'alkyle respectivement de dialkylamide de lithium sont utilisés et l'acétonitrure est transformée, à des basses températures, in situ en LiCH₂CN.

20. Procédé selon la revendication 17 à 19, caractérisé en ce que la réaction est effectuée à des températures ≤ 0°C et de préférence dans une plage de température de -20°C à -90°C.

21. Procédé selon la revendication 17 à 20, caractérisé en ce que la préparation aqueuse est effectuée de préférence à des températures ≤ 10°C et dans une plage de pH ≥ pH 6.

22. Procédé selon la revendication 17 à 21, caractérisé en ce que l'acidolyse s'effectue à des températures de +50°C dans une plage de pH ≤ 6.

23. Procédé selon les revendications 17 à 22, caractérisé en ce que l'adaptation de la gamme pH s'effectue par apport de sels par exemple NH₄Cl, NH₄OAc, NaH₂PO₄ ou par apport d'acides anorganiques comme par exemple HCl, H₃PO₄, H₂SO₄, HClO₄ ou d'acides organiques comme par exemple l'acide acétique, l'acide oxalique, l'acide sulfonique de toluène.
